# EUROPÄISCHE PATENTSCHRIFT

(11) **EP 1 010 398 B1**
(45) Veröffentlichungstag und Bekanntmachung des Hinweises auf die Patenterteilung: **29.09.2004**
(21) Anmeldenummer: 98124313.2
(22) Anmeldetag: 21.12.1998
(51) Int. Cl.: A61B 17/70

(54) **Pedikelschraube**
Pedicle screw
Vis pédiculaire

(30) Priorität: 19.12.1997 DE 19756646
(43) Veröffentlichungstag der Anmeldung: 21.06.2000
(73) Patentinhaber: MTM Medizintechnik Mauk GmbH, 22844 Norderstedt (DE)
(72) Erfinder: Hahn. Michael, 22417 Hamburg (DE)
(74) Vertreter: Heldt, Gert, Dr. Dipl.-Ing.

(56) Entgegenhaltungen:
- DE-A- 4 330 837

## Beschreibung

Die Erfindung betrifft eine Pedikelschraube mit einem in einem Wirbelkörper verankerbaren Schaftteil und einem mit einer Aufnahme für eine Fixierstange versehenen Aufnahmeteil, die über eine an einer dem Schaftteil benachbarten Unterseite des Aufnahmeteils angeordnete Nut miteinander verbindbar sind.

Fixierstangen werden unter anderem bei frischen Frakturen im thorakalen, thorakolumbalen oder lumbalen Bereich der Wirbelsäule zur kontrollierten Reposition der Fraktur sowie zur Stabilisierung der Wirbelsäule eingesetzt. Zur Befestigung der Fixierstange dienen die eingangs genannten Pedikelschrauben, die jeweils paarweise mit ihrem Schaftteil auf gegenüberliegenden Seiten des Rückenmarkskanals in Aufnahmebohrungen der Wirbelkörper (Pedikel) eingeschraubt werden, so daß ihr Aufnahmeteil dorsal übersteht. Die Verbindung der Pedikelschrauben in Längsrichtung der Wirbelsäule erfolgt mit Hilfe der Fixierstangen, die jeweils in die miteinander fluchtenden Aufnahmen zweier auf einer Seite des Rückenmarkskanals angeordneter Pedikelschrauben eingeführt werden. Die Fixierstangen selbst oder die Aufnahmeteile der Pedikelschrauben werden über die Wirbelsäule hinweg durch längenverstellbare Querverbindungen im richtigen Abstand gehalten, so daß beispielsweise eine Lordosenkorrektur ermöglicht wird.

Aus der DE-A-43 30 837 ist eine Pedikelschraube bekannt, bei der das Aufnahmeteil an seiner Unterseite eine in Längsrichtung der Aufnahme angeordnete Nut aufweist, in die das Schaftteil über eine Ausnehmung einsetzbar ist. Das Schaftteil wird in der Nut des Aufnahmeteiles durch einen in einem seitlichen Ansatz des Aufnahmeteiles angeordneten Gewindestift fixiert. Die Aufnahme ist als eine Durchgangsöffnung ausgebildet, die sich von beiden Seiten zur Mitte hin verjüngt.

Die bekannte Pedikelschraube hat sich grundsätzlich bewährt. Nachteilig bei der bekannten Pedikelschraube ist jedoch, daß der Abstand der Längsnut zur Durchgangsöffnung von der dünnsten Stelle der dazwischen liegenden Wandung abhängt. Dadurch ergibt sich eine unerwünscht hohe Bauhöhe des Aufnahmeteiles. Zudem ist die Fertigung des Aufnahmeteiles relativ aufwendig und teuer.

Aufgabe der vorliegenden Erfindung ist es daher, die bekannte Pedikelschraube so zu verbessern, daß die Bauhöhe reduziert und das Aufnahmeteil einfacher und kostengünstiger herstellbar ist.

Die Aufgabe wird erfindungsgemäß dadurch gelöst, daß die Nut mit ihrer Längsachse etwa rechtwinklig zur Längsrichtung der Aufnahme angeordnet ist.

Durch die rechtwinklige Anordnung der Nut zur Längsrichtung der Aufnahme bzw. zu deren Aufnahmeachse kann die Nut dem engsten Bereich der Durchgangsöffnung, d.h. dem Bereich mit dem meisten Material zugeordnet werden, so daß bei gleichen Festigkeitsverhältnissen die Bauhöhe reduziert werden kann. Die Nut kann zudem einfacher, d. h. kostengünstiger, hergestellt werden.

Gemäß einer weiteren bevorzugten Ausführungsform der Erfindung, weist das Aufnahmeteil einen seitlichen Ansatz mit einer in der Nut endenden Durchgangsbohrung auf, deren Bohrungsachse etwa parallel zur Schaftachse verläuft. Die Durchgangsbohrung weist ein Innengewinde auf, in das ein Gewindestift einschraubbar ist, der mit seiner dem Schaft zugewandten Stirnfläche an der Anschlagfläche des Schaftteiles anschlagbar ist. Dadurch, daß die Bohrungsachse des seitlichen Ansatzes etwa parallel zur Schaftachse verläuft, kann der Gewindestift direkt in einer Linie hinter dem Kopf des Schafteiles angeordnet werden. Dadurch wird ebenfalls die Fertigung einfacher und kostengünstiger. Durch die neue Stellung zwischen Gewindestift des Aufnahmeteiles und dem Kopf des Schaftteiles kann es zudem nicht zu einem Verklemmen beim Lösen des Gewindestiftes kommen.

Gemäß einer weiteren bevorzugten Ausführungsform der Erfindung ist der Gewindestift mit einer aus der Durchgangsbohrung in Richtung auf den Schaftteil herausragenden Konusspitze versehen, deren konisch zulaufende Seitenflächen einen äußeren Rand der Anschlagfläche im eingeschraubten Zustand des Gewindestiftes beaufschlagt. Diese Gestaltung des Gewindestiftes führt zu einer formschlüssigen Verbindung im Bereich der Konusspitze aufgrund einer Verformung, die die Konusspitze an einem die Anschlagfläche begrenzenden äußeren Rand verursacht. Aufgrund dieser Verformung entsteht zwischen der Konusspitze und dem äußeren Rand ein Formschluß, der zu einer dauerhaften und festen Verbindung zwischen dem Gewindestift und der Anschlagfläche führt. Dieser Formschluß erübrigt die Aufbringung einer hohen Formspannung im Systems durch starkes Anziehen des Gewindestiftes. Eine hohe Torsionsfestigkeit des Aufnahmeteils gegenüber dem Schaftteil verhindert, daß diese beiden Teile sich unter dem Einfluß erheblicher Kräfte voneinander lösen.

Nach einer weiteren bevorzugten Ausführungsform der Erfindung, weisen die Durchgangsbohrung und der Gewindestift ein Feingewinde auf. Der Gewindestift ist mit seiner dem Schaftteil zugewandten Stirnfläche an einer Anschlagfläche des Schaftteiles anschlagbar. Drückt der Gewindestift mit seiner Stirnfläche gegen die Anschlagfläche, blockiert sich der Gewindestift durch sein Feingewinde selbst, wenn er fest angezogen wird. Das Aufnahmeteil ist gegenüber dem Kopf des Schaftteiles solange einstellbar, solange der Gewindeschaft nicht gegen die Anschlagfläche des Schaftteiles drückt. Das Aufnahmeteil kann auf diese Weise gegenüber dem Schaftteil optimal ausgerichtet werden.

Gemäß einer weiteren bevorzugten Ausführungsform der Erfindung bestehen Gewindestift und Aufnahmeteil aus Titan. Beim festen Einschrauben des Gewindestiftes entsteht aufgrund eines Kaltschweißverhaltens ein Schweißeffekt, der eine Lockerung des Gewindestiftes verhindert.

Nach einer weiteren bevorzugten Ausführungsform der Erfindung ist die Aufnahme als eine in Längsrichtung des Aufnahmeteils etwa parallel zur Unterseite angeordnete Durchgangsöffnung, die sich von ihrer Mitte zu den außenliegenden Öffnungen hin erweitert, ausgebildet. Die Mitte der Durchgangsöffnung bildet dabei ein der Unterseite des Aufnahmeteiles benachbartes Widerlager für die in das Aufnahmeteil einschiebbare Fixierstange. Das Aufnahmeteil weist dabei an seiner der Unterseite abgewandten Oberseite zwei die der Oberseite benachbarte Wandung durchbrechende Gewindebohrungen auf, deren Längsachsen etwa parallel zur Schaftachse angeordnet sind und von dem zwischen ihnen liegenden Widerlager den gleichen Abstand aufweisen. Die Fixierstange ist durch in die Gewindebohrungen einschraubbare Stellschrauben, deren der Durchgangsöffnung zugewandten Enden die Fixierstange gegen das Widerlager drücken, gegenüber dem Aufnahmeteil um einen Kippwinkel ankippbar.

Die sich durch die Durchgangsöffnung erstreckende Fixierstange weist innerhalb des Aufnahmeteiles eine Dreipunktunterstützung auf. In der Mitte der Durchgangsöffnung liegt die Fixierstange auf dem Widerlager auf, das beispielsweise als Schneide ausgebildet sein kann. Beidseits der Schneide ist sie von den Stellschrauben beaufschlagbar. Dadurch kann das Aufnahmeteil gut gegenüber der Fixierstange ausgerichtet werden. Für die sich durch die Durchgangsöffnung erstreckende Fixierstange besteht dadurch eine Schwenkmöglichkeit von beispielsweise 30° Grad.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

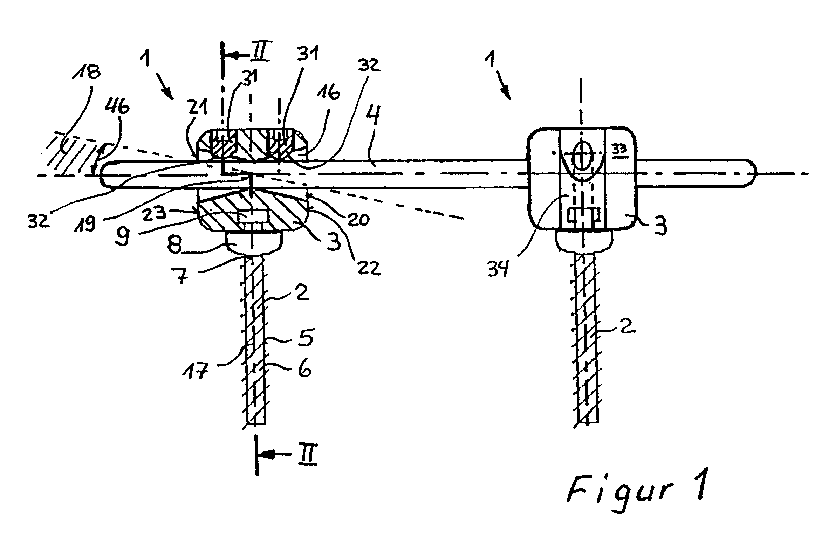

- Figur 1:: Eine Seitenansicht zweier über eine Fixierstange miteinander verbundener Pedikelschrauben, teilweise im Schnitt,

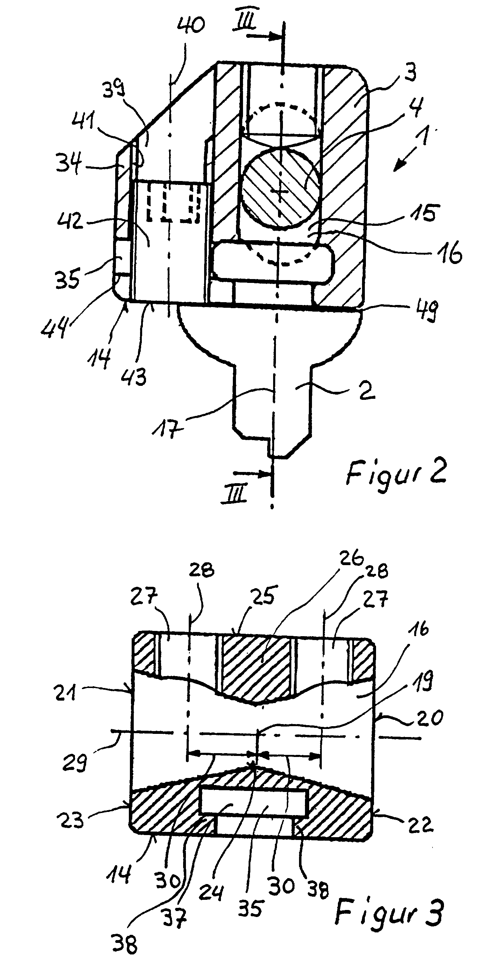

- Figur 2:: eine schematische Schnittdarstellung einer Pedikelschraube, entlang der Linie II - II von Fig. 1 geschnitten,
- Figur 3:: eine Schnittdarstellung des Aufnahmeteiles von Figur 2, entlang der Linie III - III geschnitten,

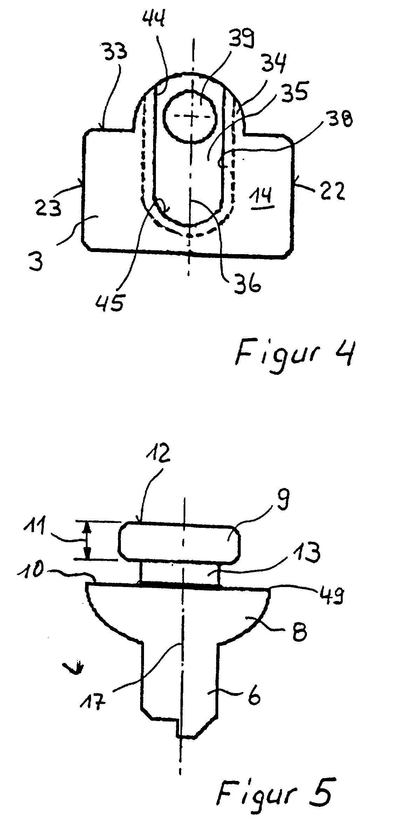

- Figur 4:: eine Untersicht unter das Aufnahmeteil von Figur 3,
- Figur 5:: eine Seitenansicht des Schaftteiles von Fig.2. und

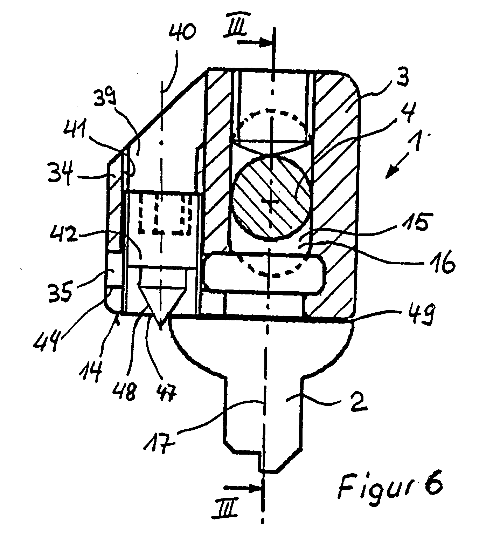

- Figur 6:: eine schematische Schnittdarstellung einer Pedikelschraube entlang der Linie II-II in Figur 1 mit einem gegenüber Figur 2 geänderten Gewindestift.

Eine Pedikelschraube (1) besteht im wesentlichen aus einem in einer Aufnahmebohrung eines Wirbelkörpers verankerbaren Schaftteil (2) und einem mit dem Schaftteil (2) verbindbaren Aufnahmeteil (3) für eine Fixierstange (4).

Das Schaftteil (2) weist einen über seine gesamte Länge mit einem Außengewinde (5) versehenen Schaft (6) auf. Der Schaft (6) weist an seinem dem Aufnahmeteil (3) zugewandten oberen Ende (7) einen radial umlaufenden Bund (8) auf, der zu dem dem Aufnahmeteil (3) benachbarten Kopfende (9) des Schaftes (6) von einer Anschlagfläche (10) begrenzt wird. Das Kopfende (9) weist in einem Abstand (11) zu seiner Stirnfläche (12) eine umlaufende Einschnürung (13) auf, die zum Schaft (6) hin von der Anschlagfläche (10) begrenzt wird.

Das Aufnahmeteil (3) weist in seiner Längsrichtung eine etwa parallel zu einer der Anschlagfläche (10) des Schaftteiles (2) benachbarten Unterseite (14) eine Aufnahme (15) auf, die als Durchgangsöffnung (16) ausgebildet ist. Die Durchgangsöffnung (16) des Aufnahmeteiles (3) erweitert sich mindestens in einer in der Schaftachse (17) des Schaftteiles (2) liegenden Ebene (18) von ihrer Mitte (19) zu ihren außenliegenden Öffnungen (20, 21) hin. Die erste außenliegende Öffnung (20) ist im Bereich einer ersten Stirnfläche (22) und die zweite außenliegende Öffnung (21) im Bereich einer der ersten Stirnfläche (22) gegenüberliegenden zweiten Stirnfläche (23) des Aufnahmeteiles angeordnet. Die Mitte (19) der Durchgangsöffnung (16) ist in ihrem unteren Bereich als ein der Unterseite (14) benachbartes Widerlager (24) für die in das Aufnahmeteil (3) einschiebbare Fixierstange (4) ausgebildet. Das Aufnahmeteil (3) weist an seiner der Unterseite (14) abgewandten Oberseite (25) zwei eine der Oberseite (25) benachbarte obere Wandung (26) durchbrechende Gewindebohrungen (27) auf. Die Gewindebohrungen (27) weisen ein Feingewinde auf. Die Längsachsen (28) der Gewindebohrungen (27) sind etwa parallel zur Schaftachse (17) angeordnet. Die Gewindebohrungen (27) weisen in Richtung der Aufnahmeachse (29) von dem zwischen ihnen liegenden Widerlager (24) den gleichen Abstand (30) auf. In die Gewindebohrungen (27) sind Stellschrauben (31) einschraubbar, durch deren der Durchgangsöffnung (16) zugewandtes Ende (32) die Fixierstange (4) gegen das Widerlager (24) drückbar ist. Für die Fixierstange (4) ergibt sich in dem Aufnahmeteil (3) bzw. in der Durchgangsöffnung (16) somit eine Dreipunktlagerung. Die Fixierstange (4) ist durch die Stellschrauben (31) gegenüber dem Aufnahmeteil (3) um einen Kippwinkel (46) ankippbar.

Das Aufnahmeteil (3) weist an seiner parallel zur Ebene (18) angeordneten Vorderseite (33) einen seitlichen Ansatz (34) auf. An seiner Unterseite (14) weist das Aufnahmeteil eine Nut (35) auf, die mit ihrer Längsachse (36) etwa rechtwinklig zur Aufnahmeachse (29) angeordnet ist. Die Nut (35) weist quer zu ihrer Längsachse (36) einen etwa T-förmig ausgeformten Querschnitt (37) auf, der auf das dem Aufnahmeteil (3) zugewandte Kopfende (9) des Schaftteiles (2) abgestimmt ist. Die Nut (35) weist an ihrer Innenseite einen die Nut (35) verengenden umlaufenden Steg (38) auf. Die Nut (35) ist an ihrem der Aufnahmeachse (29) abgewandten vorderen Ende (44) offen und an ihrem der Aufnahmeachse (29) benachbarten hinteren Ende (45) U-förmig ausgebildet. Das Aufnahmeteil (3) ist mit seiner Nut (35) seitlich auf das Kopfende (9) des Schaftteiles (2) aufsetzbar. Die Einschnürung (13) des Schaftteiles (2) wird dabei von dem die Nut (35) verengenden Steg (38) hintergriffen. Der seitliche Ansatz (34) weist eine in der Nut (35) endende Durchgangsbohrung (39) auf, deren Bohrungsachse (40) etwa parallel zur Schaftachse verläuft. Die Durchgangsbohrung (39) weist ein Innengewinde (41) auf, das als Feingewinde ausgebildet ist. In die Durchgangsbohrung (39) ist ein Gewindestift (42) einschraubbar. Der Gewindestift (42) ist mit seiner dem Schaftteil (2) zugewandten Stirnfläche (43) an der Anschlagfläche (10) des Schaftteiles (2) bzw. des Bundes (8) anschlagbar.

Der Gewindestift (42) kann statt der Stirnfläche (43) mit einer Konusspitze (48) versehen sein, die im eingeschraubten Zustand des Gewindestiftes (42) aus der Durchgangsbohrung (39) in Richtung auf den Schaftteil (2) herausragt. Dabei wird der Gewindestift (43) so in die Durchgangsbohrung (39) hineingeschraubt, daß die konisch zulaufende Seitenfläche (47) der Konusspitze (48) einen äußeren Rand (49) der Anschlagfläche (10) beaufschlagt. Dadurch wird der äußere Rand (49) der Anschlagfläche (10) verformt. In dieser Verformung erzeugt die Seitenfläche (47) einen Formschluß, der eine hohe Sicherheit dagegen bietet, daß aufgrund von Torsionsspannungen sich das Aufnahmeteil (3) gegenüber dem Schaftteil (2) verdreht. Diese formschlüssige Verbindung minimiert darüber hinaus eine im gesamten System bestehende Formspannung, die durch starkes Anziehen des Gewindestiftes (42) gegen die Anschlagfläche (10) entstehen könnte.

Nach dem Einschrauben des Schaftteiles (2) in eine Bohrung eines Wirbelkörpers wird das Aufnahmeteil (3) mit seiner Nut (35) seitlich über das Kopfende (9) des Schaftteiles (2) geschoben, bis das Kopfende (9) am hinteren Ende (45) der Nut (35) anschlägt. Das um die Schaftachse (17) drehbare Aufnahmeteil (3) wird ausgerichtet und durch Einschrauben des Gewindestiftes (42) in die Durchgangsbohrung (39) bis zum Anschlagen seiner Stirnfläche (43) gegen die Anschlagfläche (10) festgesetzt. Die Fixierstange (4) kann sich dabei bereits in der Durchgangsöffnung (16) des Aufnahmeteiles befinden. Die Fixierstange (4) kann aber auch nachträglich eingeführt werden. Die Fixierstange (4) liegt auf dem Widerlager (24) auf und wird durch Einschrauben der Stellschrauben (31) gegen das Widerlager (24) gedrückt. Durch unterschiedliches Einschrauben der Stellschrauben (31) läßt sich die Fixierstange (4) gegenüber der Aufnahmeachse (29) in der Ebene (18) ankippen.

## Patentansprüche

1. Pedikelschraube mit einem in einem Wirbelkörper verankerbaren Schaftteil und einem mit einer Aufnahme für eine Fixierstange versehenen Aufnahmeteil, die über eine an einer dem Schaftteil benachbarten Unterseite des Aufnahmeteils angeordneten Nut miteinander verbindbar sind, **dadurch gekennzeichnet, daß** die Nut (35) mit ihrer Längsachse (36) etwa rechtwinklig zur Längsrichtung der Aufnahme (15) angeordnet ist.

2. Pedikelschraube nach Anspruch 1, **dadurch gekennzeichnet, daß** die Nut (35) quer zu ihrer Längsachse (36) einen etwa T-förmig ausgeformten Querschnitt (37) aufweist, der auf ein dem Aufnahmeteil (3) zugewandtes Kopfende (9) des Schaftteiles (2) abgestimmt ist.

3. Pedikelschraube nach Anspruch 1 oder 2, **dadurch gekennzeichnet, daß** das Kopfende (9) des Schaftteiles (2) in einem Abstand (11) zu seiner Stirnfläche (12) eine Einschnürung (13) aufweist, die von einem die Nut (35) verengenden Steg (38) des Aufnahmeteiles (3) hintergreifbar ist.

4. Pedikelschraube nach Anspruch 3, **dadurch gekennzeichnet, daß** das Schaftteil (2) quer zu seiner Schaftachse (17) eine die Einschnürung (13) begrenzende Anschlagfläche (10) aufweist.

5. Pedikelschraube nach Anspruch 4, **dadurch gekennzeichnet, daß** die Anschlagfläche (10) als eine einem am Schaftteil (2) angeordneten Bund (8) zum Kopfende (9) hin begrenzende Fläche ausgebildet ist.

6. Pedikelschraube nach einem der Ansprüche 1 bis 5, **dadurch gekennzeichnet, daß** das Aufnahmeteil (3) einen seitlichen Ansatz (34) mit einer in der Nut (35) endenden Durchgangsbohrung (39) aufweist, deren Bohrungsachse (40) etwa parallel zur Schaftachse (17) verläuft.

7. Pedikelschraube nach Anspruch 6, **dadurch gekennzeichnet, daß** die Durchgangsbohrung(39) ein Innengewinde (41) aufweist, in das ein Gewindestift (42) einschraubbar ist.

8. Pedikelschraube nach Anspruch 7, **dadurch gekennzeichnet, daß** das Innengewinde (41) als Feingewinde ausgebildet ist.

9. Pedikelschraube nach Anspruch 8, **dadurch gekennzeichnet, daß** der Gewindestift (42) mit seiner dem Schaftteil (2) zugewandten Stirnfläche (43) an der Anschlagfläche (10) des Schaftteiles (2) anschlagbar ist.

10. Pedikelschraube nach Anspruch 8, **dadurch gekennzeichnet, daß** der Gewindestift (42) mit einer aus der Durchgangsbohrung (39) in Richtung auf den Schaftteil (2) herausragenden Konusspitze (48) versehen ist, deren konisch zulaufende Seitenflächen (47) einen äußeren Rand (49) der Anschlagfläche (10) im eingeschraubten Zustand des Gewindestiftes (42) beaufschlagt.

11. Pedikelschraube nach Anspruch 10, **dadurch gekennzeichnet, daß** der äußere Rand (49) unter dem Einfluß der Seitenfläche (47) verformbar ist.

12. Pedikelschraube nach einem der Ansprüche 6 bis 11, **dadurch gekennzeichnet, daß** die Nut (35) an ihrem der Durchgangsbohrung (39) benachbarten vorderen Ende (44) offen und das Aufnahmeteil (3) mit der Nut(35) auf das Kopfende (9) des Schaftteiles (2) seitlich aufsetzbar ist.

13. Pedikelschraube nach Anspruch 12, **dadurch gekennzeichnet, daß** die Nut (35) an dem dem vorderen Ende (44) gegenüberliegenden hinteren Ende (45) geschlossen und das Kopfende (9) des Schaftteiles (2) quer zur Schaftachse (17) U-förmig von der Nut (35) umgreifbar ist.

14. Pedikelschraube nach einem der Ansprüche 1 bis 13, **dadurch gekennzeichnet, daß** die Aufnahme (15) als eine in Längsrichtung des Aufnahmeteiles (3) etwa parallel zur Unterseite (14) angeordnete Durchgangsöffnung (16) ausgebildet ist.

15. Pedikelschraube nach Anspruch 14, **dadurch gekennzeichnet, daß** die Durchgangsöffnung (16) von ihrer Mitte (19) zu den außenliegenden Öffnungen (20, 21) hin sich erweitert.

16. Pedikelschraube nach Anspruch 14 oder 15, **dadurch gekennzeichnet, daß** die Mitte (19) der Durchgangsöffnung (16) in ihrem unteren Bereich als ein der Unterseite (14) benachbartes Widerlager (24) für die in das Aufnahmeteil (3) einschiebbare Fixierstange (4) ausgebildet ist.

17. Pedikelschraube nach einem der Ansprüche 14 bis 16, **dadurch gekennzeichnet, daß** das Aufnahmeteil (3) an seiner der Unterseite (14) abgewandten Oberseite (25) zwei die der Oberseite (25) benachbarte Wandung (26) durchbrechende Gewindebohrungen (24) aufweist.

18. Pedikelschraube nach Anspruch 17, **dadurch gekennzeichnet, daß** die Gewindebohrungen (27) ein Feingewinde aufweisen.

19. Pedikelschraube nach Anspruch 17 oder 18, **dadurch gekennzeichnet, daß** die Längsachsen (28) der Gewindebohrungen (27) etwa parallel zur Schaftachse (17) angeordnet sind.

20. Pedikelschraube nach einem der Ansprüche 17 bis 19, **dadurch gekennzeichnet, daß** die Gewindebohrungen (27) von dem zwischen ihnen liegenden Widerlager (24) den gleichen Abstand (30) aufweisen.

21. Pedikelschraube nach einem der Ansprüche 17 bis 20, **dadurch gekennzeichnet, daß** in die Gewindebohrungen (27) Stellschrauben (31) einschraubbar sind, durch deren der Durchgangsöffnung (16) zugewandtes Ende (32) die Fixierstange (4) gegen das Widerlager (24) drückbar ist.

22. Pedikelschraube nach Anspruch 21, **dadurch gekennzeichnet, daß** die Fixierstange (4) durch die Stellschrauben (31) gegenüber dem Aufnahmeteil (3) um einen Kippwinkel (46) ankippbar sind.

23. Pedikelschraube nach einem der Ansprüche 7 bis 22, **dadurch gekennzeichnet, daß** der Gewindestift (42) aus Titan ausgebildet ist.

24. Pedikelschraube nach einem der Ansprüche 1 bis 23, **dadurch gekennzeichnet, daß** das Aufnahmeteil (3) aus Titan ausgebildet ist.

25. Pedikelschraube nach einem der Ansprüche 21 bis 24, **dadurch gekennzeichnet, daß** die Stellschrauben (31) aus Titan ausgebildet sind.

## Claims

1. A pedicle screw comprising a shank portion which can be anchored in a vertebra and a receiving portion provided with a receiving means for a fixing rod, which portions can be connected together by way of a groove arranged at an underside of the receiving portion, said underside being adjacent to the shank portion, **characterised in that** the groove (35) is arranged with its longitudinal axis (36) approximately at a right angle to the longitudinal direction of the receiving means (15).

2. A pedicle screw according to claim 1 **characterised in that** transversely to its long axis (36) the groove (35) has a cross-section (37) which is shaped approximately as a T-shape and which is matched to a head end (9) of the shank portion (2), which head end is towards the receiving portion (3).

3. A pedide screw according to claim 1 or claim 2 **characterised in that** the head end (9) of the shank portion (2) has at a spacing (11) relative to its end face (12) a constriction (13) behind which can engage a bar (38) of the receiving portion (3), which bar narrows the groove (35).

4. A pedicle screw according to claim 3 **characterised in that** transversely to its shank axis (17) the shank portion (2) has an abutment surface (10) which delimits the constriction (13).

5. A pedicle screw according to claim 4 **characterised in that** the abutment surface (10) is in the form of a surface which towards the head end (9) delimits a shoulder (8) arranged on the shank portion (2).

6. A pedicle screw according to one of claims 1 to 5 **characterised in that** the receiving portion (3) has a lateral projection (34) with a through bore (39) which terminates in the groove (35) and the axis (40) of which extends substantially parallel to the shank axis (17).

7. A pedicle screw according to claim 6 **characterised in that** the through bore (39) has a female screwthread (41) into which a screwthreaded pin (42) can be screwed.

8. A pedicle screw according to claim 7 **characterised in that** the female screwthread (41) is in the form of a fine screwthread.

9. A pedicle screw according to claim 8 **characterised in that** the screwthreaded pin (42) can bear with its end face (43) that is towards the shank portion (2) against the abutment surface (10) of the shank portion (2).

10. A pedicle screw according to claim 8 **characterised in that** the screwthreaded pin (42) is provided with a conical tip (48) which protrudes out of the through bore (39) in a direction towards the shank portion (2) and the conically converging side surfaces (47) of which act on an outer edge (45) of the abutment surface (10) in the screwed-in condition of the screwthreaded pin (42).

11. A pedicle screw according to claim 10 **characterised in that** the outer edge (49) is deformable under the influence of the side surface (47).

12. A pedicle screw according to one of claims 6 to 11 **characterised in that** the groove (35) is open at its front end (44) adjacent to the through bore (39) and the receiving portion (3) can be laterally fitted with the groove (35) on to the head end (9) of the shank portion (2).

13. A pedicle screw according to claim 12 **characterised in that** the groove (35) is closed at the rear end (45) that is opposite to the front end (44) and the head end (9) of the shank portion (2) can be embraced in a U-shape by the groove (35) transversely with respect to the shank axis (17).

14. A pedicle screw according to one of claims 1 to 13 **characterised in that** the receiving means (15) is in the form of a through opening (16) arranged in the longitudinal direction of the receiving portion (3) substantially parallel to the underside (14).

15. A pedide screw according to claim 14 **characterised in that** the through opening (16) is enlarged from its centre (19) towards the outwardly disposed openings (20, 21).

16. A pedicle screw according to claim 14 or claim 15 **characterised in that** the centre (19) of the through opening (16) in the lower region thereof is in the form of a support (24) adjacent to the underside (14) for the fixing rod (4) which can be inserted into the receiving portion (3).

17. A pedicle screw according to one of daims 14 to 16 **characterised in that** at its top side (25) remote from the underside (14) the receiving portion (3) has two screwthreaded bores (24) which aperture the wall (26) adjacent to the top side (25).

18. A pedicle screw according to claim 17 **characterised in that** the screwthreaded bores (27) have a fine screwthread.

19. A pedicle screw according to claim 17 or claim 18 **characterised in that** the longitudinal axes (28) of the screwthreaded bores (27) are arranged substantially parallel to the shank axis (17).

20. A pedicle screw according to one of claims 17 to 19 **characterised in that** the screwthreaded bores (27) are at the same spacing (30) from the support (24) which is between them.

21. A pedide screw according to one of claims 17 to 20 **characterised in that** setscrews (31) can be screwed into the screwthreaded bores (27), wherein the end (32) of the setscrews, which is towards the through opening (16), can press the fixing rod (4) against the support (24).

22. A pedicle screw according to claim 21 **characterised in that** the fixing rod (4) can be tilted through a tilt angle (46) relative to the receiving portion (3) by the setscrews (31).

23. A pedicle screw according to one of claims 7 to 22 **characterised in that** the screwthreaded pin (42) is made of titanium.

24. A pedicle screw according to one of claims 1 to 23 **characterised in that** the receiving portion (3) is made from titanium.

25. A pedicle screw according to one of claims 1 to 24 **characterised in that** the setscrews (31) are made from titanium.

## Revendications

1. Vis pédiculaire avec une partie tige destinée à être ancrée dans une vertèbre et une partie réceptacle avec un logement pour une tige de s'immobilisation qui peuvent être liées l'une à l'autre par l'intermédiaire d'une rainure aménagée dans une face inférieure de la partie réceptrice voisine de la partie tige, **caractérisée en ce que** la rainure (35) est disposée avec son axe longitudinal orienté sensiblement perpendiculairement à la direction longitudinale du logement (15).

2. Vis pédiculaire selon la revendication 1, **caractérisée en ce que** la rainure (35), transversalement à son axe longitudinal (36), présente une section (37) sensiblement en forme de T, adaptée pour recevoir une extrémité formant tête (9) de la partie tige (2) tournée vers la partie réceptacle.

3. Vis pédiculaire selon la revendication 1 ou 2, **caractérisée en ce que** l'extrémité formant tête (9) de la partie tige (2), à une distance (11) de sa face frontale (12), comporte un rétrécissement (13) dans lequel peut pénétrer une cloison (38) de la partie réceptacle (3), qui diminue la largeur de la rainure (35)

4. Vis pédiculaire selon la revendication 3, **caractérisée en ce que** la partie tige (2), transversalement à l'axe de tige (17), présente une surface de butée (10) qui délimite le rétrécissement (13).

5. Vis pédiculaire selon la revendication 4, **caractérisée en ce que** la surface de butée (10) est réalisée sous la forme d'une surface limitant un collet (8) sur la partie tige (2), côté extrémité formant tête.

6. Vis pédiculaire selon une des revendications 1 à 5, **caractérisée en ce que** la partie réceptacle (3) comporte une saillie latérale (34) avec un trou traversant (39) qui débouche dans la rainure (35) et dont l'axe (40) est sensiblement parallèle à l'axe de tige (17).

7. Vis pédiculaire selon la revendication 6, **caractérisée en ce que** le trou traversant (39) est pourvu d'un filetage intérieur (41) dans lequel une vis sans tête (42) peut être vissée.

8. Vis pédiculaire selon la revendication 7, **caractérisée en ce que** le filetage intérieur (41) est un filetage fin.

9. Vis pédiculaire selon la revendication 8, **caractérisée en ce que** la vis sans tête (42), avec sa face frontale (43) tournée vers la partie tige (2) peut être amenée en butée contre la surface de butée (10) de la partie tige (2).

10. Vis pédiculaire selon la revendication 8, **caractérisée en ce que** la vis sans tête (42) est pourvue d'une pointe conique (48) qui fait saillie hors du trou traversant (39) en direction de la partie tige (2), dont la surface latérale (47) conique agit sur un bord extérieur (49) de la surface de butée (10), dans la position vissée de la vis sans tête (42).

11. Vis pédiculaire selon la revendication 10, **caractérisée en ce que** le bord extérieur (49) peut être déformé sous l'action de la surface latérale (47).

12. Vis pédiculaire selon une des revendications 6 à 11, **caractérisée en ce que** la rainure (35) est ouverte à son extrémité antérieure (44) voisine du trou traversant (39) et la partie réceptacle (3) portant la rainure (35) peut être enfilée latéralement sur l'extrémité formant tête (9) de la partie tige (2).

13. Vis pédiculaire selon la revendication 12, **caractérisée en ce que** la rainure (35) est fermée à son extrémité postérieure (45), opposée à l'extrémité antérieure (44), et que l'extrémité formant tête (9) de la partie tige (2) peut être entourée en U par la rainure (35), transversalement à l'axe de tige (17).

14. Vis pédiculaire selon une des revendications 1 à 13, **caractérisée en ce que** le logement (15) est conformé en trou traversant (16) qui s'étend dans la direction longitudinale de la partie réceptacle (3), sensiblement parallèlement à la face inférieure (14).

15. Vis pédiculaire selon la revendication 14, **caractérisée en ce que** le trou traversant (16), à partir de son milieu (19), s'évase en direction des ouvertures (20, 21) extérieures.

16. Vis pédiculaire selon la revendication 14 ou 15, **caractérisée en ce que** le milieu (19) du trou traversant (16) dans sa partie inférieure est conformé en contre-appui (24) voisin de la face inférieure (14) pour la tige d'immobilisation (4) enfilée dans la partie réceptacle (3).

17. Vis pédiculaire selon une des revendications 14 à 16, **caractérisée en ce que** la partie réceptacle (3), dans sa face supérieure (25) éloignée de la face inférieure (14), présente deux trous filetés (24) qui traversent la paroi (26) voisine de la face supérieure (25).

18. Vis pédiculaire selon la revendication 17, **caractérisée en ce que** les trous filetés (27) sont pourvus de filetages fins.

19. Vis pédiculaire selon la revendication 17 ou 18, **caractérisée en ce que** les axes longitudinaux (28) des trous filetés (27) sont sensiblement parallèles à l'axe de tige (17).

20. Vis pédiculaire selon une des revendications 17 à 19, **caractérisée en ce que** les trous filetés (27) sont disposés à la même distance (30) du contre-appui (24) situé entre deux.

21. Vis pédiculaire selon une des revendications 17 à 20, **caractérisée en ce que** des vis de réglage (31) peuvent être vissées dans les trous filetés (27), vis par l'extrémité (32) tournée vers le trou traversant (16) desquelles la tige d'immobilisation (4) peut être pressée contre le contre-appui (24).

22. Vis pédiculaire selon la revendication 21, **caractérisée en ce que** la tige d'immobilisation (4) peut basculer d'un angle de basculement (46) par rapport à la partie réceptacle (3) sous l'action des vis de réglage (31).

23. Vis pédiculaire selon une des revendications 7 à 22, **caractérisée en ce que** la vis sans tête (42) est en titane.

24. Vis pédiculaire selon une des revendications 1 à 23, **caractérisée en ce que** la partie réceptacle (3) est en titane.

25. Vis pédiculaire selon une des revendications 21 à 24, **caractérisée en ce que** les vis de réglage (31) sont en titane.
